# EUROPEAN PATENT APPLICATION

(11) **EP 1 844 782 A1**
(43) Date of publication of application: **17.10.2007**
(21) Application number: 06384004.5
(22) Date of filing: 27.03.2006
(51) Int. Cl.: A61K 31/529, A61P 25/00, A61P 25/04, A61P 25/08, A61P 25/14, A61P 25/28

(54) **Use of 4,9-anhydro-tetrodotoxin for the treatment of diseases related to the voltage-gated sodium channel subunit Nav1.6**

(71) Applicant: Wex Pharmaceuticals, Inc, Vancouver, BC V6C 1G8 (CA)
(72) Inventor: Buschmann, Helmut, H, Dr., 08960 Sant Just Desvern (ES); Vela Hemandez, Jose Miguel, 08041 Barcelona (ES); Schreibmayer, Wolfgang, c/o Medizinische Universität Graz, A-8010, Graz (AT)
(74) Representative: Peters, Hajo

(57) **Abstract**

The present invention refers to the use of 4,9-anhydro-tetrodotoxin for the treatment of diseases related to the voltage-gated sodium channel a subunit Naᵥ1.6.

## Description

### Field of the invention

The present invention refers to the use of 4,9-anhydro-tetrodotoxin for the treatment of diseases related to the voltage-gated sodium channel α subunit Naᵥ1.6.

### Background of the invention

Voltage-gated sodium channels are encoded by a family of ten structurally-related genes that are expressed in spatially and temporally distinct patterns, mainly in excitable tissues. They underlie electrical signaling in nerve and muscle. It has long been known that sodium channel blockers are anaesthetics as well as powerful analgesics when delivered at low concentrations. In addition, there are indications that sodium channel blockers may also be useful in the treatment of cardiac arrhythmias, epilepsy, neurodegeneration, affective disorders and schizophrenia (Wood and Boorman, 2005). As more information is available about the subtypes of sodium channels and their distribution, new therapeutic opportunities are suggested.

Increased knowledge of the molecular diversity of sodium channels, their distribution and functions has highlighted the interest in the discovery of subtype-specific channel blockers. Here we describe 4,9-anhydro-tetrodotoxin (described also as 4,9-anhydro-TTX) as a new selective blocker of the voltage-gated sodium channel α subunit Naᵥ1.6. This channel has been variously designated NaCh6, Cerill and PN4; although the current recommended nomenclature is Naᵥ1.6 and thus is used throughout the description.

The voltage-gated sodium channel α subunit Naᵥ1.6 is quite specifically involved in a number of diseases or symptoms. These include pathophysiologal symptoms of the injured nervous system such as demyelination, and neuroinflammatory disorders, where 4,9-anhydro TTX thus could have a direct neuroprotective effect on axons, and would thus also cover multiple sclerosis and (experimental) autoimmune encephalitis (EAE). On the other hand the diseases or symptoms related to the voltage-gated sodium channel α subunit Naᵥ1 .6 also include movement disorders including tremor, ataxia, dystonia and paralysis, where 4,9-anhydro TTX thus could inhibit hyperactivity affecting the CNS (i..e. epilepsy) and locomotor apparatus. In addition neuropathic pain or neuropathic pain-related hyperalgesia and allodynia are also falling under this group. Especially multiple sclerosis is a very severe disease where treatment is far from satisfying and thus it was an underlying object of this to identify drug for the treatment of diseases related to the voltage-gated sodium channel α subunit Naᵥ 1.6, especially multiple sclerosis.

4,9-anhydro-tetrodotoxin is a selective blocker of the voltage-gated sodium channel α subunit Naᵥ 1.6.

Thus, the present invention relates to the use of 4,9-anhydro-tetrodotoxin, optionally in the form of its racemate, pure stereoisomers, especially enantiomers or diastereomers or in the form of mixtures of stereoisomers, especially enantiomers or diastereomers, in any suitable mixing ratio; in neutral form, in the form of an acid or base or in form of a salt, especially a physiologically acceptable salt, or in form of a solvate, especially a hydrate for the production of a medicament for the treatment of a disease/symptom related to the voltage-gated sodium channel α subunit Naᵥ1.6.

4,9-anhydro-tetrodotoxin is inhibiting the neuronal Naᵥ1.6 in a nanomolar range and all the other subtypes either not or only in a micromolar range.

"Disease/Symptom related to the voltage-gated sodium channel α subunit Naᵥ1.6" are defined as a disease/symptom in which the voltage-gated sodium channel α subunit Naᵥ1.6 can act in a protective or ameliorating way. Specifically the following diseases or symptoms are included:
- pathophysiologal symptoms of the injured nervous system
   o demyelination
   o neuroinflammatory disorders
   o multiple sclerosis
   o (experimental) autoimmune encephalitis (EAE).
- hyperactivity affecting the CNS
   o epilepsy
- hyperactivity affecting the locomotor apparatus
- movement disorders
   o tremor,
   o ataxia,
   o dystonia
   o paralysis,
- neuropathic pain
   o neuropathic pain-related hyperalgesia
   o neuropathic pain-related allodynia
   o or diabetic neuropathy.

In addition 4,9 anhydro-TTX could have a direct neuroprotective effect on axons and thus another preferred aspect of the invention is use of 4,9-anhydro-tetrodotoxin, optionally in the form of its racemate, pure stereoisomers, especially enantiomers or diastereomers or in the form of mixtures of stereoisomers, especially enantiomers or diastereomers, in any suitable mixing ratio; in neutral form, in the form of an acid or base or in form of a salt, especially a physiologically acceptable salt, or in form of a solvate, especially a hydrate for the production of a medicament for the a neuroprotective effect.

The term "salt" according to this invention is to be understood as meaning any form of the active compound according to the invention in which this compound assumes an ionic form or is charged and - if applicable - is also coupled with a counter-ion (a cation or anion). By this are also to be understood complexes of the active compound with other molecules and ions, in particular complexes which are complexed via ionic interactions. As preferred examples of salts this includes the acetate, mono-trifluoracetate, acetate ester salt, citrate, formate, picrate, hydrobromide, monohydrobromide, monohydrochloride or hydrochloride.

The term "physiologically acceptable salt" in the context of this invention is understood as meaning a "salt" (as defined above) of at least one of the compounds according to the invention which are physiologically tolerated by humans and/or mammals.

The term "solvate" according to this invention is to be understood as meaning any form of the active compound according to the invention which has another molecule (most likely a polar solvent) attached to it via non-covalent bonding. Examples of solvates include hydrates and alcoholates, e.g. methanolate.

The term "treatment" or "to treat" in the context of this specification means administration of a compound or formulation according to the invention to prevent, ameliorate or eliminate one or more symptoms associated with (diseases related to) the voltage-gated sodium channel α subunit Naᵥ1.6.

Furthermore, the terms "to treat" or "treatment" according to this invention include the treatment of symptoms associated with (diseases related to) the voltage-gated sodium channel α subunit Naᵥ1.6., the prevention or the prophylaxis of the symptoms of associated with (diseases related to) the voltage-gated sodium channel α subunit Naᵥ1.6., the prevention or prophylaxis causing the symptoms of associated with (diseases related to) the voltage-gated sodium channel α subunit Naᵥ1.6., as well as the prevention or the prophylaxis of the consequences causing the symptoms.

According to the various embodiments, the 4,9-anhydro-TTX or the pharmaceutical compositions comprising it, may be administered, in unit dosage form, intestinally, enterally, parenterally or topically, orally, subcutaneously, intranasally, by inhalation, by oral absorption, intravenously, intramuscularly, percutaneously, intraperitoneally, rectally, intravaginally, transdermally, sublingually, buccally, orally transmucosally. Administrative dosage forms may include the following: tablets, capsules, dragees, lozenges, patches, pastilles, gels, pastes, drops, aerosols, pills, powders, liquors, suspensions, emulsions, granules, ointments, creams, suppositories, freeze-dried injections, injectable compositions, in food supplements, nutritional and food bars, syrups, drinks, liquids, cordials etc, which could be regular preparation, delayed-released preparation, controlled-released preparation and various micro-granule delivery system, in food supplements, nutritional and food bars, syrups, drinks, liquids, cordials. In case of tablet, various carriers known in the art may be used, e.g. dilutent and resorbent such as starch, dextrin, calcium sulfate, kaolin, microcrystalline cellulose, aluminium silicate, etc; wetting agent and adhesives such as water, glycerin, polyethylene glycol, ethanol, propanol, starch mucilage, dextrin, syrup, honey, glucose solution, acacia, gelatin, carboxymethylcellulose sodium, shellac, methylcellulose, potassium phosphate, polyvinylpyrrolidone, etc; disintegrating agent, such as dried starch, alginate, agar powder, laminaran, sodium bicarbonate and citric acid, calcium carbonate, polyoxyethylene sorbitol aliphatic ester, lauryl sodium sulfate, methylcellulose, ethylcellulose, lactose, sucrose, maltose, mannitol, fructose, various disaccharides and polysaccharides etc; disintegration inhibiting agent, such as sucrose, tristearin, cacao butter, hydrogenated oil, etc; absorption accelerator, such as quaternary ammonium salt, lauryl sodium sulfate, etc; lubricant, such as talc, silica, corn starch, stearate, boric acid, fluid wax, polyethylene, etc. The tablet may be further formulated into coated tablet, e.g. sugar-coated tablet, film-coated tablet, enteric-coated tablet, or double-layer tablet and multi-layer tablet. In the case of pill, various carriers known in the art may be used, e.g. dilutent and resorbent, such as glucose, lactose, starch, cacao butter, hydrogenated vegetable oil, polyvinylpyrrolidone, kaolin, talc, etc; adhesives, such as acacia, bassora gum, gelatin, ethanol, honey, liquid sugar, rice paste or flour paste, etc; disintegrating agent, such as agar powder, dried starch, alginate, lauryl sodium sulfate, methylcellulose, ethylcellulose. In case of suppository, various carriers known in the art may be used, e.g. polyethylene, lecithin, cacao butter, higher alcohols, esters of higher alcohols, gelatin, semi-synthetic glyceride, etc. In the case of capsule, it may be prepared by mixing said sodium channel blockers as active ingredient with the above mentioned carriers, followed by placing the mixture into a hard gelatin capsule or soft capsule. Also, said sodium channel blockers may be applied in the following dosage forms: microcapsules, suspension in an aqueous phase, hard capsule, or injection. In the case of injection, such as liquor, emulsion, freeze-dried injection, and suspension, all the dilutents common in the art may be used, e.g. water, ethanol, polyethylene glycol, propylene glycol, oxyethylated isostearyl alcohol, polyoxidated isostearyl alcohol, polyoxyethylene sorbitol aliphatic ester, etc. In addition, in order to obtain isotonic injection, a suitable amount of sodium chloride, glucose or glycerin may be added into the preparation, as well as regular cosolvent, buffer, pH adjusting agent, etc. In addition, coloring agent, antiseptic, perfume, correctives, food sweetening agent or other materials may be added to the pharmaceutical preparation if necessary.

In certain embodiments a formulation or pharmaceutical composition according to the invention contains the active ingredient, 4,9-anhydro-TTX as well as optionally at least one auxiliary material and/or additive and/or optionally another active ingredient.

In certain embodiments the auxiliary material and/or additive can be specifically selected from conserving agents, emulsifiers and/or carriers for parenteral application. The selection of these auxiliary materials and/or additives and of the amounts to be used depends upon how the pharmaceutical composition is to be applied. Examples include here especially parenteral like intravenous subcutaneous or intramuscular application formulations but which could also be used for other administration routes.

In alternative embodiments the 4,9-anhydro-TTX may be administered in a schedule of one, two, three, four, five, six, seven, eight, nine, ten, eleven, twelve, thirteen, fourteen, fifteen, sixteen, seventeen, eighteen, nineteen, twenty or more doses per day, alone or in combination with other medications, over range of time periods including but not limited to periods of one, two, three, four, five, six, seven, eight, nine, ten, eleven, twelve, thirteen, fourteen, sixteen, eighteen, twenty, twenty four, thirty, or more days; or over a period of one, two, three, four, five, six, seven, eight, nine, ten, eleven, twelve, thirteen, fourteen, sixteen, eighteen, twenty, twenty four, thirty, thirty six, forty eight, sixty, seventy two, eighty four or more months.

In alternative embodiments the maximum daily dose of 4,9-anhydro-TTX sodium channel blocker may be up to about 10*µ*g, up to about 50*µ*g, up to about 100,*u*g, up to about 144*µ*g, up to about 150*µ*g, up to about 300*µ*g, up to about 500*µ*g, up to about 750*µ*g, up to about 1000*µ*g, up to about 1250*µ*g, up to about 1500*µ*g, up to about 1750µg, up to about 2000µg or more. In particular embodiments the sodium channel blocker may be administered in an amount ranging between 5 and 4000µg/day, or in ranges between 10 and 2000µg/day, 10 and 1000µg a day, 10 and 750µg a day, 10 and 500 µg a day, 10 and 400 µg a day, 10 and 300 µg a day, 10 and 200 µg a day, or 10 and 100 µg/day.

In particular embodiments the daily applied dose may be from about 10 to about 160*µ*g, about 10 to about 140*µ*g, about 10 to about 120*µ*g, about 10 to about100 *µ*g, about 10 to about 90*µ*g, about 10 to about 80*µ*g, about 10 to about 70*µ*g, about 10 to about 60*µ*g, about 10 to about 50 *µ*g, about 10 to about 40*µ*g, about 10 to about 30*µ*g, or 1 to 20*µ*g. In other embodiments the daily dosage of 4,9-anhydro-TTX may be about 0.1 to about 40*µ*g per kilogram of body weight, about 1 to about 35*µ*g per kilogram of body weight, about 5 to about 30*µ*g per kilogram of body weight, about 10 to about 30*µ*g per kilogram of body weight, about 15 to about 30*µ*g per kilogram of body weight, about 10 to about 35*µ*g per kilogram of body weight, or about 20 to about 40*µ*g per kilogram of body weight. The unit dose may be within a range of about 5µg to about 2000*µ*g and may be about 5 to about 10*µ*g, about 10 to about 15*µ*g, about 15 to about 20*µ*g, about 20 to about 25*µ*g, about 25 to about 30 *µ*g, about 30 to about 40*µ*g, about 40*µ*g to about 50*µ*g, about 50*µ*g to about 75*µ*g, about 75 to about 100*µ*g, about 100 to about 150*µ*g, about 150 to about 200*µ*g, about 200 to about 250*µ*g, about 250 to about 500*µ*g, about 500 to about 1000*µ*g, about 1000 to about 1500*µ*g or about 1500 to about 2000*µ*g or more than 2000*µ*g.

In some embodiments the dose administered is between 10 and 4000 µg/day of 4,9-anhydro-tetrodotoxin, its derivatives or its analogues, especially the dose of 4,9-anhydro-tetrodotoxin administered is normally between 10 and 4000 µg/day or - given the likely twice per day treatment - between 5 to 2000 µg each given dose, sometimes preferably between 250 and 1000 µg each given dose, sometimes preferably between 25 and 50 µg each given dose depending on the route of administration.

In some embodiments the effectiveness of a course of treatment of one, two, three, four, five or more doses or one, two or three days may last for up to about five, ten, fifteen, twenty, twenty five or thirty. In some embodiments dosing is only performed once every day or once every two, three, four, five, six, seven, eight, nine, ten, eleven, twelve, thirteen, fourteen, sixteen, eighteen, twenty, twenty four, thirty or more days.

According to the present disclosure the dosage of 4,9-anhydro-TTX depends on a variety of factors, including the nature and severity of the diseases, the sex, age, weight and individual reaction of the subject, the particular compound employed, the route and frequency of administration, etc. 4,9-anhydro-TTX or the pharmaceutical compositions comprising it may be administered in single or divided dosage form, e.g. one to four doses per day. Those skilled in the art will readily understand and implement the changes to the treatment methods exemplified herein that are necessary or desirable to reflect varied therapeutic requirements.

A substance named as an "active ingredient" will have a purity of at least 97%. For example, a formulation said to have "500 µg of 4,9-anhydro-TTX as the active ingredient" may contain as much as 15 µg of an impurity.

In a preferred embodiment of the use according to the invention the disease/symptom related to the voltage-gated sodium channel α subunit Naᵥ1.6 is selected from
pathophysiologal symptoms of the injured nervous system, demyelination, neuroinflammatory disorders, multiple sclerosis, (experimental) autoimmune encephalitis (EAE), hyperactivity affecting the CNS, epilepsy, hyperactivity affecting the locomotor apparatus, movement disorders, tremor, ataxia, dystonia, paralysis, neuropathic pain, neuropathic pain-related hyperalgesia or neuropathic pain-related allodynia or diabetic neuropathy.

In an embodiment neuropathic pain is disclaimed from the diseases/symptoms related to the voltage-gated sodium channel α subunit Naᵥ1.6.

In an embodiment pain is disclaimed from the diseases/symptoms related to the voltage-gated sodium channel α subunit Naᵥ1.6.

In an embodiment central-nervously derived neuropathic pain is disclaimed from the diseases/symptoms related to the voltage-gated sodium channel α subunit Naᵥ1.6. Optionally this disclaimer also includes central-nervously derived neuropathic pain which is allodynia and/or hyperalgesia.

In an embodiment peripheral-nervously derived neuropathic pain is disclaimed from the diseases/symptoms related to the voltage-gated sodium channel α subunit Naᵥ1.6. Optionally this disclaimer also includes peripheral-nervously derived neuropathic pain which is allodynia and/or hyperalgesia.

In an embodiment non-cancer derived neuropathic pain is disclaimed from the diseases/symptoms related to the voltage-gated sodium channel α subunit Naᵥ1 .6. Otionally this disclaimer also includes non-cancer derived neuropathic pain which is allodynia and/or hyperalgesia.

In another preferred embodiment of the use according to the invention the disease/symptom related to the voltage-gated sodium channel α subunit Naᵥ1.6 is selected from
pathophysiologal symptoms of the injured nervous system, especially demyelination and/or neuroinflammatory disorders, more especially multiple sclerosis and (experimental) autoimmune encephalitis (EAE).

In another preferred embodiment of the use according to the invention the disease/symptom related to the voltage-gated sodium channel α subunit Naᵥ1.6 is selected from
hyperactivity affecting the CNS, especially epilepsy, and/or hyperactivity affecting the locomotor apparatus, especially movement disorders, more especially tremor, ataxia, dystonia or paralysis.

In another preferred embodiment of the use according to the invention the disease/symptom related to the voltage-gated sodium channel α subunit Naᵥ1.6 is selected from
neuropathic pain, neuropathic pain-related hyperalgesia or neuropathic pain-related allodynia or diabetic neuropathy.

### The diseases selected are identified on the fllowing grounds:

### Demyelination and neuroinflammation

Changes in the expression of sodium channels occur in demyelinated axons in multiple esclerosis (MS), with Naᵥ1.6 confined to nodes of Ranvier in controls but with diffuse distribution along extensive regions of demyelinated axons within acute MS plaques (Craner et al., 2004a). Naᵥ1.6 is also known to accumulate along degenerating axons in demyelinated regions of the CNS in mice with experimental autoimmune encephalitis (EAE), an experimental model of MS (Craner et al., 2004b). Co-localization of sodium channel Naᵥ1.6 and the sodium-calcium exchanger at sites of axonal injury in the spinal cord in EAE and MS has been associated with axonal degeneration: Nav1.6 is known to produce a persistent sodium current and the Na⁺/Ca²⁺ exchanger can be driven by persistent sodium current to import damaging levels of calcium into axons (Craner et al., 2004a,b). In addition, sodium channels contribute to activation of microglia and macrophages in EAE and acute MS lesions. In particular, a robust increase of sodium channel Naᵥ1.6 expression has been found in activated microglia and macrophages in EAE and MS. Treatment with the sodium channel blocker phenytoin ameliorates the inflammatory cell infiltrate in EAE by 75% and TTX reduces the phagocytic function of activated rat microglia by 40%. Microglia from med mice, which lack Naᵥ1.6 channels, show a 65% reduction in phagocytic capacity compared with microglia from wildtype mice. Altogether, these findings indicate that sodium channels are important for activation and phagocytosis of microglia and macrophages in EAE and MS and suggest that, in addition to a direct neuroprotective effect on axons, blockade of the Naᵥ1.6 sodium channel may ameliorate neuroinflammatory disorders via anti-inflammatory mechanisms (Craner et al., 2005).

In addition, endogenous Schwann cells can remyelinate demyelinated spinal cord axons and establish and maintain nodes of Ranvier on central axons for over one year, and these nodes exhibit an apparently normal distribution of sodium channels, with Naᵥ1.6 the predominant subtype of sodium channel present at such nodes at all stages of their development (Black et al., 2006). Promotion of remyelination of central axons in the demyelinated spinal cord by transplanted olfactory ensheathing cells also results in clustering of Naᵥ1.6 at nodes of remyelinated axons, recapitulating the distribution of these channels within mature nodes of uninjured axons and restoring the impulse conduction in central demyelinated axons (Sasaki et al., 2006).

### Pain

Naᵥ1.6 is abundantly expressed in spinal cord, dorsal root ganglia (DRG) and peripheral nerve, key anatomic elements involved in pain transmission (Caldwell et al., 2000; Krzemien et al., 2000; Dietrich et al., 1998). Localization of Naᵥ1.6 in presynaptic terminals in the spinal cord, as well as at the nodes of Ranvier in peripheral nerves suggest its involvement in sensory (nociceptive) processing, since it was found in all types of DRG neurons as well as in the superficial laminae of the spinal dorsal horn (Tzoumaka et al., 2000). In this way, a significant increase in transcription of mRNA and translation of Naᵥ1 .6 protein has been found within DRG neurons in painful peripheral neuropathy in the streptozotocin model of diabetes. Importantly, Naᵥ1.6 channels produce both fast transient currents and smaller persistent currents that are generated closer to resting potential. Because these persistent currents can contribute to intrinsic burst activity, the upregulation of Naᵥ1.6 channels would be expected to contribute to hyperexcitability in diabetic neurons and thus to neuropathic pain-related hyperalgesia and allodynia (Craner et al., 2002).

Naᵥ1.6 is the major sodium channel isoform at nodes of Ranvier in myelinated axons and it is distributed along unmyelinated C-fibers of sensory neurons. In the same way, mitogen-activated protein kinases (MAPKs) are expressed by neurons in some painful conditions and are activated after injury, for example, after sciatic nerve transection and inflammation. CNS neurons express both Naᵥ1.6 and p38 MAPK. In addition, spinal nerve ligation causes activation of p38 MAPK in microglia of the spinal cord, a cell type in which Naᵥ1.6 is expressed (Craner et al., 2005). Interestingly, the sodium channel Naᵥ1.6 and p38 MAP kinase colocalize in rat brain tissue and activated p38alpha phosphorylates L1 of Naᵥ1.6, specifically at serine 553. Activation of p38 in the neuronal ND7/23 cell line transfected with Naᵥ1.6 leads to a significant reduction in the peak Naᵥ1.6 current amplitude, without a detectable effect on gating properties (Wittmack et al., 2005), suggesting that regulation of Naᵥ1.6 activity by phosphorylation may act as an endogenous mechanism inhibiting pain-related hyperexcitability. In this way, phosphorylation of Naᵥ1.6 channels in painful diabetic neuropathy has been described (Hong et al., 2004).

### Hyperactivity

Movement disorders including tremor, ataxia, dystonia and paralysis have been observed in mice with mutations of the SCN8A gene coding Naᵥ1.6 (Smith and Goldin, 1999; Meisler et al., 2001). In particular, the mouse mutant medJ contains a splice site mutation in the neuronal sodium channel SCN8A that results in a very low level of expression of Nav1.6 (Sprunger et al., 1999) and this results in severe motor disturbances accompanied by delayed maturation of nodes of Ranvier, slowed nerve conduction velocity, reduced muscle mass and reduction of brain metabolic activity (Keamey et al., 2002; Hamann et al., 2003). Altogether, it is suggested that inhibition of Naᵥ1.6-mediated currents could inhibit hyperactivity affecting the CNS (i..e. epilepsy) and locomotor apparatus.

### One of the big advantages of the use according to the invention is the reduction of side effects.

At least nine different voltage-gated sodium channels have been identified in mammals. Their involvement on particular functions depends on their specific tissue or cellular distributions. Thus, the main advantage of using subtype-specific sodium channel blockers comes from the possibility to restrict the inhibitory effects on electrical excitability to particular organs, tissues and cells expressing the targeted channel. This allows the possibility to therapeutically modulate particular functions involving electrical transmission through the specific targeted channel, without affecting other functions involving other channels and thus reducing side effects.

As an example, in contrast to non-selective blockers, cardiac side effects are not expected by 4,9-anhydro-TTX as the Nav1.5 sodium channel expressed by cardiac muscle (Lei et al., 2004) is not affected. Respect to TTX, that blocks Naᵥ1.1, Naᵥ1.2, Naᵥ1.3, Naᵥ1.4, Naᵥ1.6 and Naᵥ 1.7 (but not Naᵥ1.5, Naᵥ1.8 and Naᵥ1.9), selective blocking of Naᵥ1.6 by 4,9-anhydro-TTX is expected to be devoid of side effects dependent on direct actions on skeletal musculature as the skeletal muscle-specific Naᵥ1.4 channel (Hudson et al., 1995) is not affected. Similarly, undesired effects on the CNS mediated by blocking the brain subtypes Naᵥ1.1 and Naᵥ1.2 would be avoided using 4,9-anhydro-TTX instead of TTX.

### One of the big advantages of the use according to the invention is the specificity of actions.

The involvement of a particular channel on a particular function depends on their specific distribution but also on their intrinsic properties. Thus, the therapeutic opportunities of a selective subtype-specific sodium channel blocker largely depend on the distribution and functional properties of the targeted channel.

The SCN8A gene encodes the voltage-gated sodium channel Naᵥ1.6, which is widely expressed in neurons of the CNS and PNS. In fact, Naᵥ1.6 is abundant at the axon initial segment and is the major sodium channel isoform at nodes of Ranvier (Caldwell et al., 2000). It is present in the soma of small sensory neurons in dorsal root ganglion (DRG) and along their myelinated and unmyelinated fibers within the sciatic nerve (Caldwell et al., 2000; Krzemien et al., 2000; Tzoumaka et al., 2000). Recently, Naᵥ1.6 has been shown to accumulate along degenerating axons in demyelinated regions of the CNS in mice with experimental autoimmune encephalitis (EAE) and in patients with multiple sclerosis (MS) (Craner et al., 2004a,b). Additionally, Naᵥ1.6 has been shown to be significantly upregulated in activated microglia and macrophages in EAE and in acute MS lesions (Craner et al., 2005). Thus, Naᵥ1.6 appears to play an important role in normal axonal conduction and may significantly contribute to the pathophysiology of the injured nervous system. (Wittmack et al., 2005).

Naᵥ1.6 channels produce a persistent current in addition to fast activating and inactivating TTX-sensitive currents (Herzog et al., 2003). The rapidly activating and inactivating Naᵥ1.6 current is characterized by fast recovery from inactivation and this appears to poise Naᵥ1.6 for high-frequency firing of myelinated fibers that do not, however, fire in response to sustained slow depolarizations. Nodes of Ranvier are the axonal regions without myelin and glial cell wrapping, which are essential for the efficient conduction of action potentials. Naᵥ1.6 is the predominant isoform at the nodes of Ranvier in both sensory and motor neurons of the adult CNS and PNS (Caldwell et al., 2000). Interestingly, Naᵥ1.6 shows use-dependent potentiation during a rapid train of depolarizations. Rapid stimulation accelerated a slow phase of activation in the Naᵥ1.6 channel that had fast inactivation removed, resulting in faster channel activation. As repetitive depolarizations accelerated the activation kinetics of Naᵥ1.6 and this channels is thus more resistant to inactivation that other channels, Naᵥ1.6 might be specialized to more faithfully propagate high-frequency firing at nodes of Ranvier compared to other sodium channels (Zhou and Goldin, 2004).

The ability to generate resurgent currents is also a differential feature of Naᵥ1.6 sodium channels. Resurgent sodium currents are an unusual type of sodium current that reactivate during mild repolarizations following depolarization to positive potentials and may be critical in determining the firing patterns of neurons. While classic sodium channel tail currents activate instantaneously and decay rapidly, resurgent currents show much slower kinetics.

Naᵥ1.6 currents activate and inactivate during depolarization, as well as reactivate during repolarization from positive potentials, producing a "resurgent" current. This reopening of channels not only generates inward current after each action potential, but also permits rapid recovery from inactivation. DRG neurons, particularly large diameter DRG neurons, posses Naᵥ1.6 channels and it is known that these neurons show channels that open transiently during recovery from inactivation, generating a resurgent sodium current that flows immediately following action potentials. Interestingly, DRG neurons from wild-type mice, but not from Naᵥ1.6-null mice can produce resurgent currents, indicating that Naᵥ1.6 channels underlie the generation of these currents (Cummins et al., 2005). Similarly, in Purkinje neurons from mutant med mice that lack expression of the SCN8A gene, which encodes the Naᵥ1.6 protein, transient sodium current inactivates more rapidly than in wild-type cells, and resurgent current is nearly abolished. Regular, high-frequency firing was slowed in med Purkinje neurons. In addition the loss of Naᵥ1.6-specific kinetics slowed simulated spontaneous activity. Together, the data indicate that Nav1.6 promote and accelerate firing (Khaliq et al., 2003). The association of Nav1.6 α subunits with β4 subunits has been suggested to be needed for the generation of resurgent currents (Bean, 2005).

### References

o Black JA, Waxman SG, Smith KJ. Remyelination of dorsal column axons by endogenous Schwann cells restores the normal pattern of Nav1.6 and Kv1.2 at nodes of Ranvier. Brain. 2006.
o Sasaki M, Black JA, Lankford KL, Tokuno HA, Waxman SG, Kocsis JD. Molecular reconstruction of nodes of Ranvier after remyelination by transplanted olfactory ensheathing cells in the demyelinated spinal cord. J Neurosci. 2006 26:1803-1812.
o Wittmack EK, Rush AM, Hudmon A, Waxman SG, Dib-Hajj SD. Voltage-gated sodium channel Nav1.6 is modulated by p38 mitogen-activated protein kinase. J Neurosci. 2005 25:6621-30.
o Zhou W, Goldin AL. Use-dependent potentiation of the Nav1.6 sodium channel. Biophys J. 2004 87(6):3862-72.
o Craner MJ, Damarjian TG, Liu S, Hains BC, Lo AC, Black JA, Newcombe J, Cuzner ML, Waxman SG. Sodium channels contribute to microglia/macrophage activation and function in EAE and MS. Glia. 2005 49(2):220-9.
o Lei M, Jones SA, Liu J, Lancaster MK, Fung SS, Dobrzynski H, Camelliti P, Maier SK, Noble D, Boyett MR. Requirement of neuronal- and cardiac-type sodium channels for murine sinoatrial node pacemaking. J Physiol. 2004 559(Pt 3):835-48.
o Craner MJ, Newcombe J, Black JA, Hartle C, Cuzner ML, Waxman SG. Molecular changes in neurons in multiple sclerosis: altered axonal expression of Nav1.2 and Nav1.6 sodium channels and Na+/Ca2+ exchanger. Proc Natl Acad Sci U S A. 2004a 101(21):8168-73.
o Craner MJ, Hains BC, Lo AC, Black JA, Waxman SG. Co-localization of sodium channel Nav1.6 and the sodium-calcium exchanger at sites of axonal injury in the spinal cord in EAE. Brain. 2004b 127(Pt 2):294-303.
o Herzog RI, Cummins TR, Ghassemi F, Dib-Hajj SD, Waxman SG. Distinct repriming and closed-state inactivation kinetics of Nav1.6 and Nav1.7 sodium channels in mouse spinal sensory neurons. J Physiol. 2003 551(Pt 3):741-50.
o Cummins TR, Dib-Hajj SD, Herzog RI, Waxman SG. av1.6 channels generate resurgent sodium currents in spinal sensory neurons. FEBS Lett. 2005 579(10):2166-70.
o Bean BP. The molecular machinery of resurgent sodium current revealed. Neuron. 2005 45(2):185-7.
o Khaliq ZM, Gouwens NW, Raman IM. The contribution of resurgent sodium current to high-frequency firing in Purkinje neurons: an experimental and modeling study. J Neurosci. 2003 23(12):4899-912.
o Keamey JA, Buchner DA, De Haan G, Adamska M, Levin SI, Furay AR, Albin RL, Jones JM, Montal M, Stevens MJ, Sprunger LK, Meisler MH. Molecular and pathological effects of a modifier gene on deficiency of the sodium channel Scn8a (Na(v)1.6). Hum Mol Genet. 2002 11 (22):2765-75.
o Hamann M, Meisler MH, Richter A. Motor disturbances in mice with deficiency of the sodium channel gene Scn8a show features of human dystonia. Exp Neurol. 2003 184(2):830-8.
o Meisler MH, Keamey J, Escayg A, MacDonald BT, Sprunger LK. Sodium channels and neurological disease: insights from Scn8a mutations in the mouse. Neuroscientist. 2001 7(2):136-45.
o Tzoumaka E, Tischler AC, Sangameswaran L, Eglen RM, Hunter JC, Novakovic SD. Differential distribution of the tetrodotoxin-sensitive rPN4/NaCh6/Scn8a sodium channel in the nervous system. J Neurosci Res. 2000 60(1):37-44.
o Smith MR, Goldin AL. A mutation that causes ataxia shifts the voltage-dependence of the Scn8a sodium channel. Neuroreport. 1999 10(14):3027-31.
o Sprunger LK, Escayg A, Tallaksen-Greene S, Albin RL, Meisler MH. Dystonia associated with mutation of the neuronal sodium channel Scn8a and identification of the modifier locus Scnm1 on mouse chromosome 3. Hum Mol Genet. 1999 8(3):471-9.
o Dietrich PS, McGivern JG, Delgado SG, Koch BD, Eglen RM, Hunter JC, Sangameswaran L. Functional analysis of a voltage-gated sodium channel and its splice variant from rat dorsal root ganglia. J Neurochem. 1998 70(6):2262-72.
o Craner MJ, Klein JP, Renganathan M, Black JA, Waxman SG. Changes of sodium channel expression in experimental painful diabetic neuropathy. Ann Neurol. 2002 52(6):786-92.
o Hong S, Morrow TJ, Paulson PE, Isom LL, Wiley JW. Early painful diabetic neuropathy is associated with differential changes in tetrodotoxin-sensitive and -resistant sodium channels in dorsal root ganglion neurons in the rat. J Biol Chem. 2004 279(28):29341-50.
o Wood JN, Boorman J. Voltage-gated sodium channel blockers; target validation and therapeutic potential. Curr Top Med Chem. 2005 5(6):529-37.
o Hudson AJ, Ebers GC, Bulman DE. The skeletal muscle sodium and chloride channel diseases. Brain. 1995 118 (Pt 2):547-63.
o Caldwell JH, Schaller KL, Lasher RS, Peles E, Levinson SR. Sodium channel Nav1.6 is localized at nodes of Ranvier, dendrites, and synapses. Proc Natl Acad Sci U S A. 2000 97(10):5616-20.
o Krzemien DM, Schaller KL, Levinson SR, Caldwell JH. Immunolocalization of sodium channel isoform NaCh6 in the nervous system. J Comp Neurol. 2000 420(1):70-83.

In another preferred embodiment of the use according to the invention the 4,9, anhydro-tetrodotoxin is used in an amount of 0.1 to 5.0 µg/kg per dose, especially between 0.1 to 1.5 µg/kg per dose

In another preferred embodiment of the use according to the invention the 4,9, anhydro-tetrodotoxin is used in an amount between 10 µg/day and 4 mg/day, especially between 20 and 100 µg/day.

In another preferred embodiment of the use according to the invention the 4,9-anhydro-tetrodotoxin is isolated from a biological source, preferably from fish, especially puffer fish.

In another preferred embodiment of the use according to the invention the 4,9-anhydro-tetrodotoxin is synthetic.

Included in this invention is especially also a method of treatment of a patient or a mammal, including men, suffering from a disease related to the voltage-gated sodium channel α subunit Naᵥ1.6 using 4,9-anhydro-TTX, optionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate,. It is also preferred if in the method of treatment 4,9-anhydro-tetrodotoxin is used in an amount between 10 *µ*g/day and 4 mg/day, especially in a dose of 0.1-1 *µ*g/kg, is isolated from a biological source, preferably from fish, especially puffer fish, or is synthesized/synthetic.

### Other aspect of the invention:

Another aspect of the invention is the use of a sodium channel blocker, including especially tetrodotoxin and/or its derivatives, optionally in the form of its racemate, pure stereoisomers, especially enantiomers or diastereomers or in the form of mixtures of stereoisomers, especially enantiomers or diastereomers, in any suitable mixing ratio; in neutral form, in the form of an acid or base or in form of a salt, especially a physiologically acceptable salt, or in form of a solvate, especially a hydrate for the production of a medicament for the treatment of a disease/symptom related to the voltage-gated sodium channel α subunit Naᵥ1.6.

"Disease/Symptom related to the voltage-gated sodium channel α subunit Naᵥ1.6" are defined as a disease/symptom in which the voltage-gated sodium channel α subunit Naᵥ1.6 can act in a protective or ameliorating way. Specifically the following diseases or symptoms are included:
- pathophysiologal symptoms of the injured nervous system
   o demyelination
   o neuroinflammatory disorders
   o multiple sclerosis
   o (experimental) autoimmune encephalitis (EAE).
- hyperactivity affecting the CNS
   o epilepsy
- hyperactivity affecting the locomotor apparatus
- movement disorders
   o tremor,
   o ataxia,
   o dystonia
   o paralysis,
- neuropathic pain
   o neuropathic pain-related hyperalgesia
   o neuropathic pain-related allodynia
   o or diabetic neuropathy.

In a preferred embodiment of the use according to the invention the disease/symptom related to the voltage-gated sodium channel α subunit Naᵥ 1.6 is selected from
pathophysiologal symptoms of the injured nervous system, demyelination, neuroinflammatory disorders, multiple sclerosis, (experimental) autoimmune encephalitis (EAE), hyperactivity affecting the CNS, epilepsy, hyperactivity affecting the locomotor apparatus, movement disorders, tremor, ataxia, dystonia, paralysis, neuropathic pain, neuropathic pain-related hyperalgesia, neuropathic pain-related allodynia, or diabetic neuropathy.

In an embodiment neuropathic pain is disclaimed from the diseases/symptoms related to the voltage-gated sodium channel α subunit Naᵥ1.6.

In an embodiment pain is disclaimed from the diseases/symptoms related to the voltage-gated sodium channel α subunit Naᵥ1.6.

In an embodiment central-nervously derived neuropathic pain is disclaimed from the diseases/symptoms related to the voltage-gated sodium channel α subunit Naᵥ1.6. Optionally this disclaimer also includes central-nervously derived neuropathic pain which is allodynia and/or hyperalgesia.

In an embodiment peripheral-nervously derived neuropathic pain is disclaimed from the diseases/symptoms related to the voltage-gated sodium channel α subunit Naᵥ1.6. Optionally this disclaimer also includes peripheral-nervously derived neuropathic pain which is allodynia and/or hyperalgesia.

In an embodiment non-cancer derived neuropathic pain is disclaimed from the diseases/symptoms related to the voltage-gated sodium channel α subunit Naᵥ1.6. Otionally this disclaimer also includes non-cancer derived neuropathic pain which is allodynia and/or hyperalgesia.

In another preferred embodiment of the use according to the invention the disease/symptom related to the voltage-gated sodium channel α subunit Naᵥ1.6 is selected from
pathophysiologal symptoms of the injured nervous system, especially demyelination and/or neuroinflammatory disorders, more especially multiple sclerosis and (experimental) autoimmune encephalitis (EAE).

In another preferred embodiment of the use according to the invention the disease/symptom related to the voltage-gated sodium channel α subunit Naᵥ1.6 is selected from
hyperactivity affecting the CNS, especially epilepsy, and/or hyperactivity affecting the locomotor apparatus, especially movement disorders, more especially tremor, ataxia, dystonia or paralysis.

In another preferred embodiment of the use according to the invention the disease/symptom related to the voltage-gated sodium channel α subunit Naᵥ1.6 is selected from
neuropathic pain, neuropathic pain-related hyperalgesia, neuropathic pain-related allodynia, or diabetic neuropath.

According to the various embodiments, the sodium channel blockers such as TTX or STX, their analogues/derivatives or the pharmaceutical compositions comprising them, may be administered, in unit dosage form, intestinally, enterally, parenterally or topically, orally, subcutaneously, intranasally, by inhalation, by oral absorption, intravenously, intramuscularly, percutaneously, intraperitoneally, rectally, intravaginally, transdermally, sublingually, buccally, orally transmucosally. Administrative dosage forms may include the following: tablets, capsules, dragees, lozenges, patches, pastilles, gels, pastes, drops, aerosols, pills, powders, liquors, suspensions, emulsions, granules, ointments, creams, suppositories, freeze-dried injections, injectable compositions, in food supplements, nutritional and food bars, syrups, drinks, liquids, cordials etc, which could be regular preparation, delayed-released preparation, controlled-released preparation and various micro-granule delivery system, in food supplements, nutritional and food bars, syrups, drinks, liquids, cordials. In case of tablet, various carriers known in the art may be used, e.g. dilutent and resorbent such as starch, dextrin, calcium sulfate, kaolin, microcrystalline cellulose, aluminium silicate, etc; wetting agent and adhesives such as water, glycerin, polyethylene glycol, ethanol, propanol, starch mucilage, dextrin, syrup, honey, glucose solution, acacia, gelatin, carboxymethylcellulose sodium, shellac, methylcellulose, potassium phosphate, polyvinylpyrrolidone, etc; disintegrating agent, such as dried starch, alginate, agar powder, laminaran, sodium bicarbonate and citric acid, calcium carbonate, polyoxyethylene sorbitol aliphatic ester, lauryl sodium sulfate, methylcellulose, ethylcellulose, lactose, sucrose, maltose, mannitol, fructose, various disaccharides and polysaccharides etc; disintegration inhibiting agent, such as sucrose, tristearin, cacao butter, hydrogenated oil, etc; absorption accelerator, such as quaternary ammonium salt, lauryl sodium sulfate, etc; lubricant, such as talc, silica, corn starch, stearate, boric acid, fluid wax, polyethylene, etc. The tablet may be further formulated into coated tablet, e.g. sugar-coated tablet, film-coated tablet, enteric-coated tablet, or double-layer tablet and multi-layer tablet. In the case of pill, various carriers known in the art may be used, e.g. dilutent and resorbent, such as glucose, lactose, starch, cacao butter, hydrogenated vegetable oil, polyvinylpyrrolidone, kaolin, talc, etc; adhesives, such as acacia, bassora gum, gelatin, ethanol, honey, liquid sugar, rice paste or flour paste, etc; disintegrating agent, such as agar powder, dried starch, alginate, lauryl sodium sulfate, methylcellulose, ethylcellulose. In case of suppository, various carriers known in the art may be used, e.g. polyethylene, lecithin, cacao butter, higher alcohols, esters of higher alcohols, gelatin, semi-synthetic glyceride, etc. In the case of capsule, it may be prepared by mixing said sodium channel blockers as active ingredient with the above mentioned carriers, followed by placing the mixture into a hard gelatin capsule or soft capsule. Also, said sodium channel blockers may be applied in the following dosage forms: microcapsules, suspension in an aqueous phase, hard capsule, or injection. In the case of injection, such as liquor, emulsion, freeze-dried injection, and suspension, all the dilutents common in the art may be used, e.g. water, ethanol, polyethylene glycol, propylene glycol, oxyethylated isostearyl alcohol, polyoxidated isostearyl alcohol, polyoxyethylene sorbitol aliphatic ester, etc. In addition, in order to obtain isotonic injection, a suitable amount of sodium chloride, glucose or glycerin may be added into the preparation, as well as regular cosolvent, buffer, pH adjusting agent, etc. In addition, coloring agent, antiseptic, perfume, correctives, food sweetening agent or other materials may be added to the pharmaceutical preparation if necessary.

In certain embodiments a formulation or pharmaceutical composition according to the invention contains the active ingredient (TTX, its derivatives and/or its analogues) as well as optionally at least one auxiliary material and/or additive and/or optionally another active ingredient.

In certain embodiments the auxiliary material and/or additive can be specifically selected from conserving agents, emulsifiers and/or carriers for parenteral application. The selection of these auxiliary materials and/or additives and of the amounts to be used depends upon how the pharmaceutical composition is to be applied. Examples include here especially parenteral like intravenous subcutaneous or intramuscular application formulations but which could also be used for other administration routes.

In certain embodiments routes of administration of tetrodotoxin its derivatives and its analogues can include intramuscular injection, intraveneous injection, subcutaneous injection, sublingual, bucal, patch through skin, oral ingestion, implantable osmotic pump, collagen implants, aerosols or suppository.

In alternative embodiments the sodium channel blockers such as TTX or STX, their analogues/derivatives may be administered in a schedule of one, two, three, four, five, six, seven, eight, nine, ten, eleven, twelve, thirteen, fourteen, fifteen, sixteen, seventeen, eighteen, nineteen, twenty or more doses per day, alone or in combination with other medications, over range of time periods including but not limited to periods of one, two, three, four, five, six, seven, eight, nine, ten, eleven, twelve, thirteen, fourteen, sixteen, eighteen, twenty, twenty four, thirty, or more days; or over a period of one, two, three, four, five, six, seven, eight, nine, ten, eleven, twelve, thirteen, fourteen, sixteen, eighteen, twenty, twenty four, thirty, thirty six, forty eight, sixty, seventy two, eighty four or more months.

In particular embodiments the sodium channel blockers such as TTX or STX, their analogues/derivatives may be a voltage-gated sodium channel blocker and may bind to a SS1 or SS2 a subunit of a sodium channel. The maximum daily dose of sodium channel blocker may be up to about 10*µ*g, up to about 50*µ*g, up to about 100*µ*g, up to about 144*µ*g, up to about 150*µ*g, up to about 300*µ*g, up to about 500*µ*g, up to about 750*µ*g, up to about 1000*µ*g, up to about 1250*µ*g, up to about 1500*µ*g, up to about 1750*µ*g, up to about 2000*µ*g or more. In particular embodiments the sodium channel blocker may be administered in an amount ranging between 5 and 4000 *µ*g/day, or in ranges between 10 and 2000*µ*g/day, 10 and 1000*µ*g a day, 10 and 750 *µ*g a day, 10 and 500*µ*g a day, 10 and 400*µ*g a day, 10 and 300 *µ*g a day, 10 and 200*µ*g a day, or 10 and 100 *µ*g/day.

In particular embodiments the daily applied dose may be from about 10 to about 160µg, about 10 to about 140µg, about 10 to about 120µg, about 10 to about100 µg, about 10 to about 90µg, about 10 to about 80µg, about 10 to about 70µg, about 10 to about 60 *µ*g, about 10 to about 50 *µ*g, about 10 to about 40*µ*g, about 10 to about 30*µ*g, or 1 to 20*µ*g. In other embodiments the daily dosage of the sodium channel blocker may be about 0.1 to about 40µg per kilogram of body weight, about 1 to about 35*µ*g per kilogram of body weight, about 5 to about 30*µ*g per kilogram of body weight, about 10 to about 30*µ*g per kilogram of body weight, about 15 to about 30*µ*g per kilogram of body weight, about 10 to about 35*µ*g per kilogram of body weight, or about 20 to about 40*µ*g per kilogram of body weight. The unit dose may be within a range of about 5*µ*g to about 2000*µ*g and may be about 5 to about 10*µ*g, about 10 to about 15*µ*g, about 15 to about 20*µ*g, about 20 to about 25*µ*g, about 25 to about 30 *µ*g, about 30 to about 40*µ*g, about 40µg to about 50*µ*g, about 50*µ*g to about 75*µ*g, about 75 to about 100*µ*g, about 100 to about 150*µ*g, about 150 to about 200*µ*g, about 200 to about 250*µ*g, about 250 to about 500*µ*g, about 500 to about 1000*µ*g, about 1000 to about 1500*µ*g or about 1500 to about 2000*µ*g or more than 2000*µ*g.

In some embodiments the dose administered is between 10 and 4000 µg/day of tetrodotoxin, its derivatives or its analogues, especially the dose of tetrodotoxin administered is normally between 10 and 4000 µg/day or - given the likely twice per day treatment - between 5 to 2000 µg each given dose, sometimes preferably between 250 and 1000 µg each given dose, sometimes preferably between 25 and 50 µg each given dose depending on the route of administration.

In some embodiments the effectiveness of a course of treatment of one, two, three, four, five or more doses or one, two or three days may last for up to about five, ten, fifteen, twenty, twenty five or thirty. In some embodiments dosing is only performed once every day or once every two, three, four, five, six, seven, eight, nine, ten, eleven, twelve, thirteen, fourteen, sixteen, eighteen, twenty, twenty four, thirty or more days.

According to the present disclosure, the dosage of said sodium channel blocker such as TTX or STX, their analogues/derivatives, depends on a variety of factors, including the nature and severity of the diseases, the sex, age, weight and individual reaction of the subject, the particular compound employed, the route and frequency of administration, etc. Said sodium channel blockers such as TTX or STX, their analogues/derivatives or the pharmaceutical compositions comprising them may be administered in single or divided dosage form, e.g. one to four doses per day. Those skilled in the art will readily understand and implement the changes to the treatment methods exemplified herein that are necessary or desirable to reflect varied therapeutic requirements.

A substance named as an "active ingredient" will have a purity of at least 97%. For example, a formulation said to have "500 µg of TTX as the active ingredient" may contain as much as 15 µg of anhydrotetrodotoxin as an impurity. On the other hand, a formulation said to have "500 µg of TTX and 500 µg of anhydrotetrodotoxin as active ingredients" will contain at least 485 µg of TTX and 485 µg of anhydrotetrodotoxin, but may contain as much as 30 µg of other substances as impurities of the active ingredients. Of course, substances named as other components of a formulation are not included when the purity of the active ingredient is considered.

The term "analogues" as used in this application is defined here as meaning a chemical compound that is a derivative of a compound which has similar biochemical activity to that compound. For example, "Analogues" of TTX bind to the same site on the alpha subunit of sodium channels as does TTX.

The term "derivatives" as used in this application is defined here as meaning a chemical compound having undergone a chemical derivation such as substitution or addition of a further chemical group to change (for pharmaceutical use) any of its physico-chemical properties, such as solubility or bioavailability. Derivatives include so-called prodrugs, e.g. ester and ether derivatives of an active compound that yield the active compound per se after administration to a subject.

Examples of well known methods of producing a prodrug of a given acting compound are known to those skilled in the art and can be found e.g. in Krogsgaard-Larsen et al., Textbook of Drugdesign and Discovery, Taylor & Francis (April 2002). "Sodium channel blockers" or "sodium channel blocking compounds" in the sense of this invention thus encompass any chemicals that bind selectively to the to the voltage-gated sodium channel *α* subunit Naᵥ1.6 and thereby deactivate the sodium channel. Particularly tetrodotoxin is found to possess similar pharmaceutical activity (US 6,407,088, hereby incorporated by reference).

Tetrodotoxin (alternatively in the context of this application abbreviated TTX), also known as Ti Qu Duo Xin, is an alkaloid found in puffer fish (Tetradontiae). The chemical name is Octahydro-12-(Hydroxymethyl)-2-imino-5, 9, 7, 10a-dimethano-10aH-[1,3]dioxocino[6,5-d]pyrimidine-4,7,10,11,12-pentol with a molecular formula C₁₁H₁₇N₃O₈ and a Molecular weight of 319.27. It is a potent non-protein neurotoxin and an indispensable tool for the study of neurobiology and physiology. Tetrodotoxin (TTX) is a marine organic toxin which is mainly found in testicles, ovaries, eggs, livers, spleens, eyeballs, and blood of puffer fish as well as in diverse animal species, including goby fish, newt, frogs and the blue ringed octopus and even in marine alga. Several processes for producing TTX are known. Usually TTX is extracted from marine organisms (e.g. JP 270719 Goto and Takahashi) but besides numerous others methods of synthesis are also described (and used for the preparation of tetrodotoxin in connection to this invention) in US 6,552,191, US6,478,966, US 6,562,968 or 2002/0086997, all of which are included here by reference. Tetrodotoxin is a well known compound described for example in WO02/22129 as systemically acting as analgesic. For one of the many descriptions of TTX it is recommended turn to e.g. Tu, Anthony (Ed.) Handbook of Natural Toxins, Vol. 3: Marine Toxins and Venoms, 1988, 185-210 as well as Kao (1966), Pharmacol. Rev. 18:997 - 1049 and others.

The phrase "its (tetrodoxin's) derivatives" according to this invention is defined - using the definition of US 6,030,974 (included here by reference) - as meaning amino perhydroquinazoline compounds having the molecular formula C₁₁H₁₇N₃O₈. "Tetrodoxin's derivatives" according to this invention encompasses compounds described in US 5,846,975 (included here by reference) as amino hydrogenated quinazolines and derivatives including the substances set forth from column 3 line 40 to column 6 line 40. Specifically exemplified "derivatives of tetrodotoxin" according to this invention are including but are not limited to anhydro-tetrodotoxin, tetrodaminotoxin, methoxytetrodotoxin, ethoxytetrodotoxin, deoxytetrodotoxin and tetrodonic acid, 6 epi-tetrodotoxin, 11-deoxytetrodotoxin as well as the hemilactal type TTX derivatives (e.g. 4-*epi*-TTX, 6-*epi*-TTX, 11*-deoxy-*TTX, 4*-epi-*11*-deoxy* TTX, TTX-8-*O*-hemisuccinate, chiriquitoxin, 11-*nor*-TTX-6(S)-ol, 11-*nor*-TTX-6(R)-ol, 11-*nor*-TTX-6,6-diol, 11-*oxo*-TTX and TTX-11-carboxylic acid), the lactone type TTX derivatives (e.g. 6-*epi*-TTX (lactone), 11*-deoxy-*TTX (lactone), 11-*no*r-TTX-6(S)ol (lactone), 11-*no*r-TTX-6(R)-ol (lactone), 11-*nor*-TTX-6,6-diol (lactone), 5-*deoxy*-TTX, 5,11-*dideoxy* TTX, 4-*epi*-5,11-didroxy-TTX, 1-*hydroxy*-5,11-dideoxy-TTX, 5,6,11-*trideoxy-*TTX and 4*-epi-*5,6,11*-trideoxy*-TTX) and the 4,9-anhydro type TTX analogs (e.g., 4,9*-anhydro-*6*-epi-*TTX, 4,9*-anhydro-*11*-deoxy-*TTX, 4,9-*anhydro*-TTX-8-*O-*hemisuccinate, 4,9-*anhydro*-TTX-11-*O*-hemisuccinate). The typical derivatives of TTX possess only 1/8 to 1/40 of the toxicity of TTX in mice, based upon bioassay in mice. It has been observed that these derivatives produce joint action, and do not interact adversely. Examples of TTX derivatives include novel TTX derivatives isolated from various organisms, as well as those that are partially or totally chemically synthesized (see e.g., Yotsu, M. et al. Agric. Biol. Chem., 53(3):893-895 (1989)).

"Derivatives and analogues of TTX", as referred to in the present invention, may include compounds having the general formula I 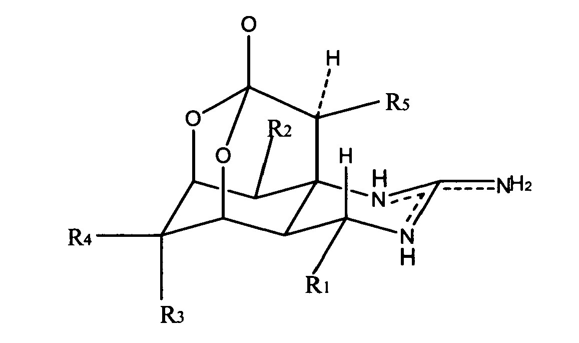 wherein, R₂ and R₅ can be selected from the group consisting of H, OH, OAC, respectively;
R₁ call be H, or an alkyl with C₁-C₄, OH, OR, OC(O)R', NH₂, NHR", NR"R"', among them R can be an alkyl with C₁-C₆, R' can be an alkyl with C₁-C₃, and R", R'" can be an alkyl with C₁-C₄, respectively;
R₃ and R₄ can be =O, or
when R₃ is H, R₄ can be selected from the group consisting of:
-ROH, and R is a branched or straight chain alkyl with C₁-C₇,
-CH(OH)NHOMe,
-NAP-gly,
-NAP-en,
-CH₂NH₂,
- CH₂NHCH₃,
-AAG,
-NMAG, and
-ANT;
when R₃ is OH or OC(O)R and R is an alkyl with C₁-C₃, R₄ can be selected from the group consisting of:
-CHO,
-CH₂-gly,
-CH₂-β-Ala,
-CH₂-Lys,
-CH₂-en,
-CH₂-NAP-Lys
-CH₂-NAP-en,
-CH(OH)CH(NH₂)COOH; and,
-NH(CH₂)nCOOH,
-NH(CH₂)ₙNH₂; and
-NH(CH₂)ₙCH(NH₂)COOH,
wherein:
n=1-6.
en is ethylene;
NAP is 4-triazo-2-nitrobenzoic amide, indicated as formula (a);
AAG is 2-triazo-O-aminobenzoic amide, indicated as formular (b);
NMAG is O-methylaminobenzoic amide, indicated as formula (c);
ANT is O-aminobenzoic amide, indicated as formula (d);

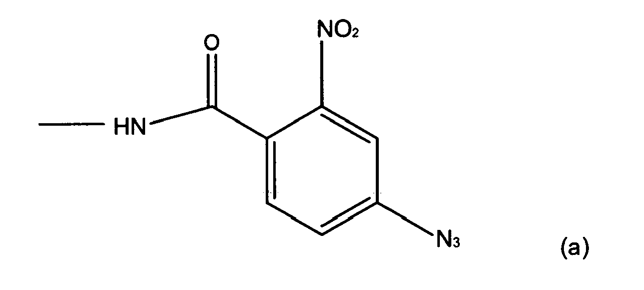

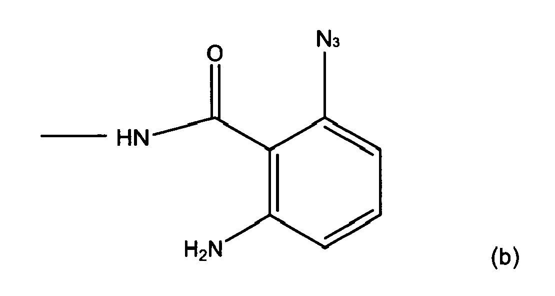

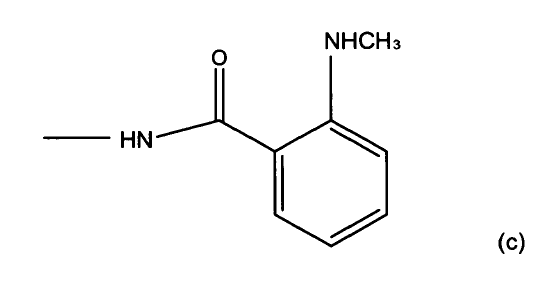

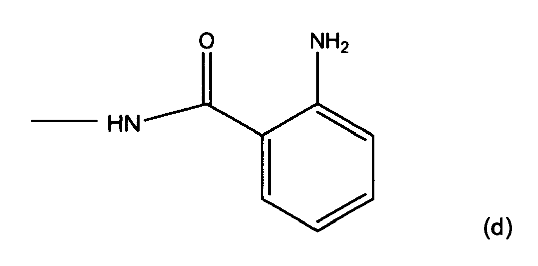

Among them, three kinds of compounds with the general formula ll, III, IV are alternative.

The amino hydrogenated quinazoline compounds and derivatives thereof are compounds having following general formula II, 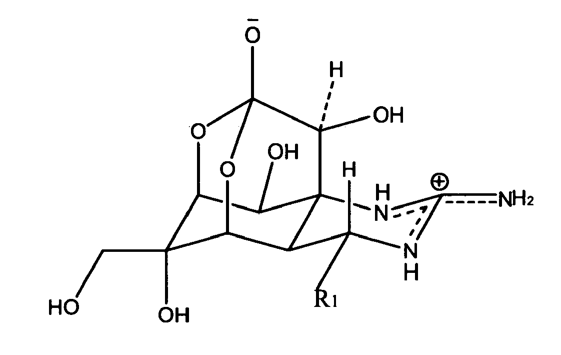 wherein: R₁ can be selected from the group consisting of OH, an alkyl or a oxyalkyl with C₁-C₄, NH₂, NHR", NR"R"', among them R" and R"' can be an alkyl with C₁ -C₄.

Among them, the more preferred compounds are:
Tetrodotoxin R₁ =OH (1);
deoxytetrodotoxin R₁=H (2);

The amino hydrogenated quiniazoline compounds and derivatives thereof are compounds having following general formula III 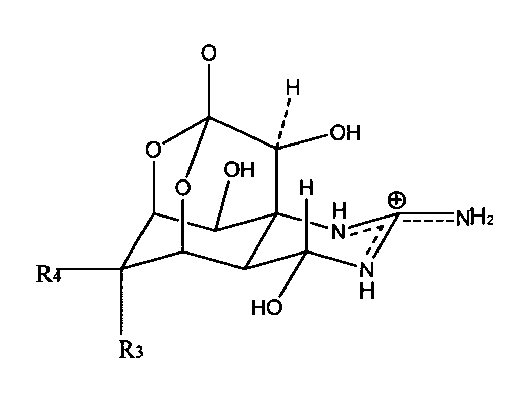 wherein:
R₃, R₄ are=O, or
when R₃ is H, R₄ is selected from the group consisting of:
CH₂OH,
CH(OH)NHOMe,
-NAP-gly,
-NAP-en,
-CH₂NH₂,
-CH₂NHCH₃,
-AAG,
-NMAG, and
-ANT.

Among them, the more preferred compounds are:
AAG-degradation Tetrodotoxin R₄=AAG (3);
NMAG-degradation Tetrodotoxin R₄=NMAG (4);
ANT-degradation Tetrodotoxin R₄=ANT (5); and,
degradation Tetrodotoxin R₃, R₄ is =O (6).

The amino hydrogenated quinazoline and their derivatives are compounds having following general formula IV, 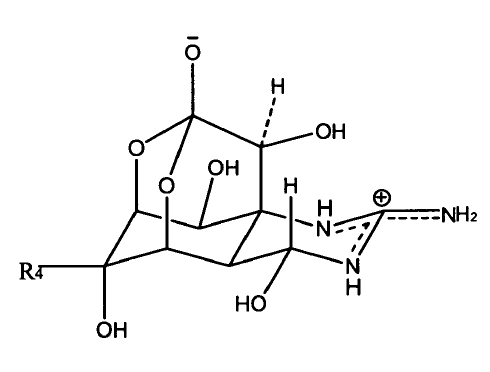 wherein, R₄ can be selected from the group consisting of:
-CHO,
-CH₂-Gly,
-CH₂-β-Ala,
-CH₂-Lys,
-CH₂-en,
-CH₂-NAP-Lys
-CH₂-NAP-en,
--CH(OH)CH(NH₂)COOH;
-NH(CH₂)₄CH(NH₂)COOH;
-NHCH₂COOH;
-NHCH₂CH₂COOH; and
-NHCH₂CH₂NH₂.

Among them, the more preferred compounds are:
oxytetrodotoxin R₄=CHO (7);
chiriquitoxin R₄=CH(OH)CH(NH₂)COOH (8);
and the compounds with the substituted groups of R₄ :
-NH(CH₂)₄ CH(NH₂)COOH (9);
-NHCH₂COOH (10);
-NHCH₂CH₂COOH (11); and,
-NHCH₂ CH₂ NH₂ (12).

Preferably, the derivatives/analogues of tetrodotoxin comprise tetrodotoxin, anhydro-tetrodotoxin, tetrodaminotoxin, methoxytetrodotoxin, ethoxytetrodotoxin, deoxytetrodotoxin, epi-tetrodotoxin and tetrodonic acid, more preferably the derivatives/analogues of tetrodotoxin a consisting of tetrodotoxin, anhydrotetrodotoxin, tetrodaminotoxin, methoxytetrodotoxin, ethoxytetrodotoxin, deoxytetrodotoxin, epi-tetrodotoxin (4- and 6-epi-tetrodotoxin) and tetrodonic acid.

Saxitoxin (STX) and its pharmacologically acceptable salts are species of 2,6-diamino -4-((aminocarbonyl)oxy) methyl-3a,4,8,9 -tetrahydro-1H, 10H- pyrrolo(1,2-c) purine -10,10-diol (3aS-(3a-a-a-4-a,10aR*)). The molecular formula of Saxitoxin is C₁₀H₁₇N₇O₄, it has a molecular weight of 299.3 and a general structure of:

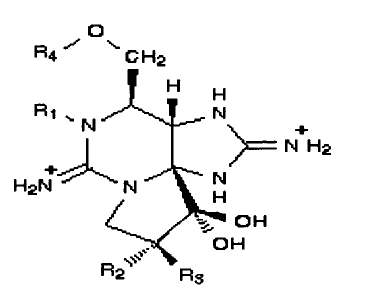

This, and its derivatives and its analogues may be used in accordance with the disclosure. Saxitoxin is readily soluble in water and can be dispersed in aerosols. It is toxic by ingestion and by inhalation, with inhalation leading to rapid respiratory collapse and death. Chemically, saxitoxin is stable, although it can be inactivated by treatment with strong alkali. It is naturally-occurring, produced by bacteria that grow in other organisms, including the dinoflagellates Gonyaulax catenella and G. tamarensis; which are consumed by the Alaskan butter clam Saxidomus giganteus and the California sea mussel, Mytilus californianeus. The toxin can be isolated from S. giganteus or M. californianeus. The first synthesis of STX was completed by Kishi and co-workers at Harvard in 1977 (J. Am. Chem. Soc. 1977, 99, 2818). A second synthesis was carried out by Jacobi and his collaborators whilst at Wesleyan University, Connecticut (J. Am. Chem. Soc. 1984, 106, 5594). A range of alternative methods for the synthesis and purification of saxitoxin will be apparent to those skilled in the art. Analogues and derivatives of saxitoxin include but are not limited to neosaxitoxin and anhydrosaxitoxin, any other biologically active variants of the above saxitoxin structure, and pharmaceutically acceptable salts thereof.

It will be appreciated that for the purposes set out herein, tetrodotoxin, saxitoxin, and their derivatives or analogues or metabolite, can be optionally in the form of their racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate.

According to the various embodiments, the sodium channel blockers such as TTX or STX, their analogues/derivatives or the pharmaceutical compositions comprising them, may be administered, in unit dosage form, intestinally, enterally, parenterally or topically, orally, subcutaneously, intranasally, by inhalation, by oral absorption, intravenously, intramuscularly, percutaneously, intraperitoneally, rectally, intravaginally, transdermally, sublingually, buccally, orally transmucosally. Administrative dosage forms may include the following: tablets, capsules, dragees, lozenges, patches, pastilles, gels, pastes, drops, aerosols, pills, powders, liquors, suspensions, emulsions, granules, ointments, creams, suppositories, freeze-dried injections, injectable compositions, in food supplements, nutritional and food bars, syrups, drinks, liquids, cordials etc, which could be regular preparation, delayed-released preparation, controlled-released preparation and various micro-granule delivery system, in food supplements, nutritional and food bars, syrups, drinks, liquids, cordials. In case of tablet, various carriers known in the art may be used, e.g. dilutent and resorbent such as starch, dextrin, calcium sulfate, kaolin, microcrystalline cellulose, aluminium silicate, etc; wetting agent and adhesives such as water, glycerin, polyethylene glycol, ethanol, propanol, starch mucilage, dextrin, syrup, honey, glucose solution, acacia, gelatin, carboxymethylcellulose sodium, shellac, methylcellulose, potassium phosphate, polyvinylpyrrolidone, etc; disintegrating agent, such as dried starch, alginate, agar powder, laminaran, sodium bicarbonate and citric acid, calcium carbonate, polyoxyethylene sorbitol aliphatic ester, lauryl sodium sulfate, methylcellulose, ethylcellulose, lactose, sucrose, maltose, mannitol, fructose, various disaccharides and polysaccharides etc; disintegration inhibiting agent, such as sucrose, tristearin, cacao butter, hydrogenated oil, etc; absorption accelerator, such as quaternary ammonium salt, lauryl sodium sulfate, etc; lubricant, such as talc, silica, corn starch, stearate, boric acid, fluid wax, polyethylene, etc. The tablet may be further formulated into coated tablet, e.g. sugar-coated tablet, film-coated tablet, enteric-coated tablet, or double-layer tablet and multi-layer tablet. In the case of pill, various carriers known in the art may be used, e.g. dilutent and resorbent, such as glucose, lactose, starch, cacao butter, hydrogenated vegetable oil, polyvinylpyrrolidone, kaolin, talc, etc; adhesives, such as acacia, bassora gum, gelatin, ethanol, honey, liquid sugar, rice paste or flour paste, etc; disintegrating agent, such as agar powder, dried starch, alginate, lauryl sodium sulfate, methylcellulose, ethylcellulose. In case of suppository, various carriers known in the art may be used, e.g. polyethylene, lecithin, cacao butter, higher alcohols, esters of higher alcohols, gelatin, semi-synthetic glyceride, etc. In the case of capsule, it may be prepared by mixing said sodium channel blockers as active ingredient with the above mentioned carriers, followed by placing the mixture into a hard gelatin capsule or soft capsule. Also, said sodium channel blockers may be applied in the following dosage forms: microcapsules, suspension in an aqueous phase, hard capsule, or injection. In the case of injection, such as liquor, emulsion, freeze-dried injection, and suspension, all the dilutents common in the art may be used, e.g. water, ethanol, polyethylene glycol, propylene glycol, oxyethylated isostearyl alcohol, polyoxidated isostearyl alcohol, polyoxyethylene sorbitol aliphatic ester, etc. In addition, in order to obtain isotonic injection, a suitable amount of sodium chloride, glucose or glycerin may be added into the preparation, as well as regular cosolvent, buffer, pH adjusting agent, etc. In addition, coloring agent, antiseptic, perfume, correctives, food sweetening agent or other materials may be added to the pharmaceutical preparation if necessary.

In certain embodiments a formulation or pharmaceutical composition according to the invention contains the active ingredient (TTX, its derivatives and/or its analogues) as well as optionally at least one auxiliary material and/or additive and/or optionally another active ingredient.

In certain embodiments the auxiliary material and/or additive can be specifically selected from conserving agents, emulsifiers and/or carriers for parenteral application. The selection of these auxiliary materials and/or additives and of the amounts to be used depends upon how the pharmaceutical composition is to be applied. Examples include here especially parenteral like intravenous subcutaneous or intramuscular application formulations but which could also be used for other administration routes.

In certain embodiments routes of administration of tetrodotoxin its derivatives and its analogues can include intramuscular injection, intraveneous injection, subcutaneous injection, sublingual, bucal, patch through skin, oral ingestion, implantable osmotic pump, collagen implants, aerosols or suppository.

In alternative embodiments the sodium channel blockers such as TTX or STX, their analogues/derivatives may be administered in a schedule of one, two, three, four, five, six, seven, eight, nine, ten, eleven, twelve, thirteen, fourteen, fifteen, sixteen, seventeen, eighteen, nineteen, twenty or more doses per day, alone or in combination with other medications, over range of time periods including but not limited to periods of one, two, three, four, five, six, seven, eight, nine, ten, eleven, twelve, thirteen, fourteen, sixteen, eighteen, twenty, twenty four, thirty, or more days; or over a period of one, two, three, four, five, six, seven, eight, nine, ten, eleven, twelve, thirteen, fourteen, sixteen, eighteen, twenty, twenty four, thirty, thirty six, forty eight, sixty, seventy two, eighty four or more months.

In particular embodiments the sodium channel blockers such as TTX or STX, their analogues/derivatives may be a voltage-gated sodium channel blocker and may bind to a SS1 or SS2 a subunit of a sodium channel. The maximum daily dose of sodium channel blocker may be up to about 10*µ*g, up to about 50*µ*g, up to about 100*µ*g, up to about 144*µ*g, up to about 150*µ*g, up to about 300*µ*g, up to about 500*µ*g, up to about 750*µ*g, up to about 1000*µ*g, up to about 1250*µ*g, up to about 1500*µ*g, up to about 1750*µ*g, up to about 2000*µ*g or more. In particular embodiments the sodium channel blocker may be administered in an amount ranging between 5 and 4000 *µ*g/day, or in ranges between 10 and 2000*µ*g/day, 10 and 1000*µ*g a day, 10 and 750*µ*g a day, 10 and 500*µ*g a day, 10 and 400 *µ*g a day, 10 and 300 *µ*g a day, 10 and 200*µ*g a day, or 10 and 100 *µ*g/day.

In particular embodiments the daily applied dose may be from about 10 to about 160*µ*g, about 10 to about 140*µ*g, about 10 to about 120*µ*g, about 10 to about100 *µ*g, about 10 to about 90*µ*g, about 10 to about 80*µ*g, about 10 to about 70*µ*g, about 10 to about 60 *µ*g, about 10 to about 50 *µ*g, about 10 to about 40*µ*g, about 10 to about 30*µ*g, or 1 to 20*µ*g. In other embodiments the daily dosage of the sodium channel blocker may be about 0.1 to about 40*µ*g per kilogram of body weight, about 1 to about 35*µ*g per kilogram of body weight, about 5 to about 30*µ*g per kilogram of body weight, about 10 to about 30*µ*g per kilogram of body weight, about 15 to about 30*µ*g per kilogram of body weight, about 10 to about 35*µ*g per kilogram of body weight, or about 20 to about 40*µ*g per kilogram of body weight. The unit dose may be within a range of about 5*µ*g to about 2000*µ*g and may be about 5 to about 10*µ*g, about 10 to about 15*µ*g, about 15 to about 20*µ*g, about 20 to about 25*µ*g, about 25 to about 30 *µ*g, about 30 to about 40*µ*g, about 40*µ*g to about 50*µ*g, about 50*µ*g to about 75*µ*g, about 75 to about 100*µ*g, about 100 to about 150*µ*g, about 150 to about 200µg, about 200 to about 250µg, about 250 to about 500*µ*g, about 500 to about 1000*µ*g, about 1000 to about 1500µg or about 1500 to about 2000µg or more than 2000µg.

In some embodiments the dose administered is between 10 and 4000 µg/day of tetrodotoxin, its derivatives or its analogues, especially the dose of tetrodotoxin administered is normally between 10 and 4000 µg/day or - given the likely twice per day treatment - between 5 to 2000 µg each given dose, sometimes preferably between 250 and 1000 µg each given dose, sometimes preferably between 25 and 50 µg each given dose depending on the route of administration.

In some embodiments the effectiveness of a course of treatment of one, two, three, four, five or more doses or one, two or three days may last for up to about five, ten, fifteen, twenty, twenty five or thirty. In some embodiments dosing is only performed once every day or once every two, three, four, five, six, seven, eight, nine, ten, eleven, twelve, thirteen, fourteen, sixteen, eighteen, twenty, twenty four, thirty or more days.

According to the present disclosure, the dosage of said sodium channel blocker such as TTX or STX, their analogues/derivatives, depends on a variety of factors, including the nature and severity of the diseases, the sex, age, weight and individual reaction of the subject, the particular compound employed, the route and frequency of administration, etc. Said sodium channel blockers such as TTX or STX, their analogues/derivatives or the pharmaceutical compositions comprising them may be administered in single or divided dosage form, e.g. one to four doses per day. Those skilled in the art will readily understand and implement the changes to the treatment methods exemplified herein that are necessary or desirable to reflect varied therapeutic requirements.

A substance named as an "active ingredient" will have a purity of at least 97%. For example, a formulation said to have "500 µg of TTX as the active ingredient" may contain as much as 15 µg of anhydrotetrodotoxin as an impurity. On the other hand, a formulation said to have "500 µg of TTX and 500 µg of anhydrotetrodotoxin as active ingredients" will contain at least 485 µg of TTX and 485 µg of anhydrotetrodotoxin, but may contain as much as 30 µg of other substances as impurities of the active ingredients. Of course, substances named as other components of a formulation are not included when the purity of the active ingredient is considered.

In a highly preferred embodiment of the invention the use according to the invention tetrodotoxin, its derivative and/or one of its analogues is used in an amount between 10 µg/day and 4 mg/day.

In a highly preferred embodiment of the invention the use according to the invention the used tetrodotoxin, its derivative or its analogue is isolated from a biological source, preferably from fish, especially puffer fish.

In a highly preferred embodiment of the invention the use according to the invention the used tetrodotoxin, its derivative or its analogue is synthesized / synthetic.

The examples and figures in the following section describing pharmacological trials are merely illustrative and the invention cannot be considered in any way as being restricted to these applications.

### Examples

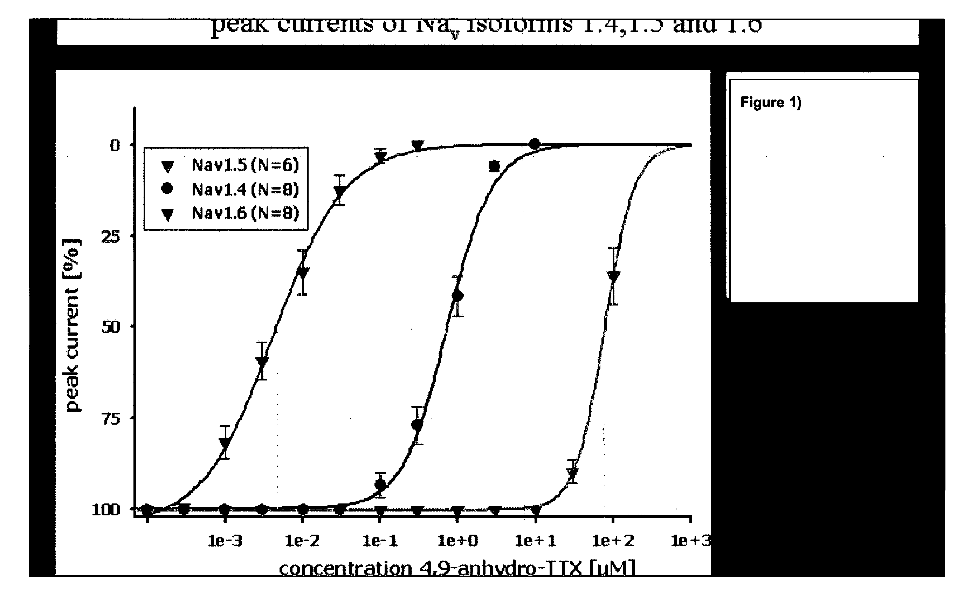

Dose response relations of 4,9-anhydro-TTX from voltage-clamp recordings of Xenopus laevis oocytes expressing Naᵥ 1.4, 1.5, 1.6 were measured. Cells were kept in ND96 medium. Rising concentrations of 4,9-anhydro-TTX were applied. Normalized mean values from at least 3 independent patches of cells. The results are shown in figure 1. The number of experiments is indicated in the figure 1.

The neuronal isoform Naᵥ 1.6 exerts IC₅₀'s in the nanomolar range, while Naᵥ 1.4 and Naᵥ 1.5 are blocked by micomolar concentrations only.

Using the same experimental procedure with TTX revealed TTX sensitive channels (Naᵥ 1.2, Naᵥ 1.3, Naᵥ 1.4, Naᵥ 1.6 and Naᵥ 1.7 and insensitive ones (Naᵥ 1.5 and Naᵥ 1.8).

## Claims

1. Use of 4,9-anhydro-TTX, optionally in the form of its racemate, pure stereoisomers, especially enantiomers or diastereomers or in the form of mixtures of stereoisomers, especially enantiomers or diastereomers, in any suitable mixing ratio; in neutral form, in the form of an acid or base or in form of a salt, especially a physiologically acceptable salt, or in form of a solvate, especially a hydrate for the production of a medicament for the treatment of a disease/symptom related to the voltage-gated sodium channel α subunit Naᵥ1.6

2. Use, according to Claim 1, in which the disease/symptom related to the voltage-gated sodium channel α subunit Naᵥ1.6 is selected from
pathophysiologal symptoms of the injured nervous system, demyelination, neuroinflammatory disorders, multiple sclerosis, (experimental) autoimmune encephalitis (EAE), hyperactivity affecting the CNS, epilepsy, hyperactivity affecting the locomotor apparatus, movement disorders, tremor, ataxia, dystonia, paralysis, neuropathic pain, neuropathic pain-related hyperalgesia, neuropathic pain-related allodynia, or diabetic neuropath.

3. Use, according to Claim 2, in which the disease/symptom related to the voltage-gated sodium channel α subunit Naᵥ1.6 is selected from
pathophysiologal symptoms of the injured nervous system, especially demyelination and/or neuroinflammatory disorders,
more especially multiple sclerosis and (experimental) autoimmune encephalitis (EAE).

4. Use, according to Claim 2, in which the disease/symptom related to the voltage-gated sodium channel α subunit Naᵥ1.6 is selected from
hyperactivity affecting the CNS, especially epilepsy, and/or
hyperactivity affecting the locomotor apparatus, especially movement disorders, more especially tremor, ataxia, dystonia or paralysis.

5. Use, according to Claim 2, in which the disease/symptom related to the voltage-gated sodium channel α subunit Naᵥ1.6 is selected from
neuropathic pain, neuropathic pain-related hyperalgesia, neuropathic pain-related allodynia, or diabetic neuropath.

6. Use of 4,9-anhydro-tetrodotoxin, optionally in the form of its racemate, pure stereoisomers, especially enantiomers or diastereomers or in the form of mixtures of stereoisomers, especially enantiomers or diastereomers, in any suitable mixing ratio; in neutral form, in the form of an acid or base or in form of a salt, especially a physiologically acceptable salt, or in form of a solvate, especially a hydrate for the production of a medicament for the a neuroprotective effect.

7. Use according to any of claims 1 to 6, **characterized in that** 4,9, anhydrotetrodotoxin is used in an amount of 0.1 to 5.0 µg/kg per dose, especially between 0.1 to 1.5 µg/kg per dose

8. Use according to any of claims 1 to 6, **characterized in that** 4,9, anhydrotetrodotoxin is used in an amount between 10 *µ*g/day and 4 mg/day, especially between 20 and 100 *µ*g/day.

9. Use according to any of claims 1 to 8, **characterized in that** 4,9-anhydrotetrodotoxin is isolated from a biological source, preferably from fish, especially puffer fish.

10. Use according to any or claims 1 to 8, **characterized in that** 4,9-anhydrotetrodotoxin is synthetic.
